(19) Europäisches Patentamt / European Patent Office / Office européen des brevets

(11) **EP 2 873 436 B1**

(12) **FASCICULE DE BREVET EUROPEEN**

(45) Date de publication et mention
de la délivrance du brevet:
**22.03.2017 Bulletin 2017/12**

(51) Int Cl.:
*A61N 1/362* (2006.01)    *A61N 1/368* (2006.01)
*A61N 1/365* (2006.01)

(21) Numéro de dépôt: **14191418.4**

(22) Date de dépôt: **03.11.2014**

(54) **Dispositif médical implantable actif à stimulation biauriculaire pour le traitement de l'insuffisance cardiaque à fraction d'éjection préservée HFpEF**

Implantierbare aktive medizinische Vorrichtung zur biaurikulären Stimulation für die Behandlung von Herzinsuffizienz mit erhaltener Auswurffraktion (HFpEF)

Active implantable medical device with bi-auricular stimulation for treating cardiac insufficiency with HFpEF preserved ejection fraction

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.11.2013 FR 1361199**

(43) Date de publication de la demande:
**20.05.2015 Bulletin 2015/21**

(73) Titulaire: **Sorin CRM SAS**
**92143 Clamart Cedex (FR)**

(72) Inventeurs:
• **Amblard, Amel**
**92290 Chatenay Malabry (FR)**
• **Limousin, Marcel**
**75014 PARIS (FR)**

(74) Mandataire: **Grünecker Patent- und Rechtsanwälte**
**PartG mbB**
**Leopoldstraße 4**
**80802 München (DE)**

(56) Documents cités:
EP-A1- 2 471 575    US-B1- 6 941 170
US-B1- 7 308 306    US-B1- 7 474 921

EP 2 873 436 B1

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle concerne plus particulièrement les dispositifs destinés à traiter l'insuffisance cardiaque (HF, *Heart Failure*), en alternative ou en complément au traitement des troubles du rythme cardiaque.

Cette thérapie vise à resynchroniser la contraction des cavités cardiaques (oreillette et ventricule, et les deux ventricules entre eux) de manière à améliorer l'état du patient par optimisation des différentes phases du cycle hémodynamique, ce cycle comprenant : pré-éjection, contraction isovolumique, éjection systolique, relaxation isovolumique, et enfin remplissage de la cavité.

La plupart de ces dispositifs mettent en oeuvre une technique dite "CRT" (*Cardiac Resynchronization Therapy*) ou "BVP" (*Bi-Ventricular Pacing*) consistant à délivrer en tant que de besoin des impulsions électriques permettant d'assurer une stimulation conjointe et permanente des deux ventricules, gauche et droit, afin de resynchroniser ces derniers.

Cette technique de resynchronisation biventriculaire ne s'adresse toutefois qu'à l'une des formes d'insuffisance cardiaque, dite "insuffisance systolique". Dans cette forme de la maladie, le muscle cardiaque est dans l'incapacité de fournir l'effort nécessaire pour assurer un débit cardiaque suffisant, et le patient présente des signes de dilatation entraînant un retard de dépolarisation du ventricule gauche. La stimulation biventriculaire CRT permet alors de resynchroniser les ventricules et de rendre plus homogène la contraction cardiaque.

L'autre forme d'insuffisance cardiaque, dite "insuffisance diastolique" ou "à fraction d'éjection préservée" (HFpEF, *Heart Failure with preserved Ejection Fraction*) ne présente pas de caractère de désynchronisation des ventricules, mais provient d'un défaut de remplissage du ventricule gauche.

Une stimulation CRT biventriculaire sera donc sans effet dans ce cas de figure.

Or cette pathologie touche près de 40 % des insuffisants cardiaques, et il n'existe pas de traitement efficace connu pour y remédier.

Cette forme de pathologie peut être chez certains patients la conséquence d'un trouble de conduction au niveau des oreillettes (bloc interauriculaire), qui induit un retard de la dépolarisation, et donc de la contraction, de l'oreillette gauche (OG) par rapport à l'oreillette droite (OD). En revanche, comme les voies de conduction atrioventriculaires ne sont pas altérées, la dépolarisation et la contraction des deux ventricules droit (VD) et gauche (VG) surviennent dans un délai normal, sans désynchronisation VD-VG. C'est entre la contraction de l'oreillette gauche et celle du ventricule gauche que le bloc interauriculaire OD-OG engendre un mauvais séquencement OG-VG : le retard de la contraction de l'oreillette gauche fait que celle-ci se contracte pratiquement en même temps que le ventricule gauche, et donc ne peut pas remplir correctement sa fonction et contribuer au remplissage actif du ventricule gauche.

Pour traiter cette pathologie d'insuffisance cardiaque à fraction d'éjection préservée, il a été proposé une technique *d'overdriving* auriculaire, décrite par exemple dans le US 7 474 921 B1, consistant à stimuler l'oreillette gauche en permanence à une fréquence légèrement supérieure à la fréquence spontanée du rythme sinusal (donc du rythme de l'oreillette droite), de manière à provoquer systématiquement une dépolarisation prématurée de l'oreillette gauche et rétablir de ce fait une séquence OG-VG quasiment normale.

Plus précisément, dans cette technique connue, le dispositif évalue régulièrement le rythme spontané du patient et applique une séquence d'impulsions de stimulation à un rythme légèrement plus rapide, arbitrairement programmé pour provoquer une prématurité de l'ordre de 50 à 100 ms par rapport à un intervalle de couplage auriculaire correspondant au rythme sinusal spontané. Après plusieurs cycles à ce rythme accéléré, la fréquence est ralentie progressivement jusqu'à réapparition de l'activité spontanée, puis le processus *d'overdriving* est réitéré de la même façon et ainsi de suite.

Dans la présente description, on entendra par "intervalle de couplage auriculaire" ou "couplage auriculaire" l'intervalle de temps séparant deux évènements auriculaires consécutifs spontanés (en rythme sinusal, également désigné "intervalle PP") ou stimulés.

La fréquence de stimulation appliquée varie donc en continu entre des valeurs où elle est trop rapide (période *d'overdriving*) ou trop lente (période de réapparition du rythme spontané, avec désynchronisation OD-VD), sans véritable contrôle de l'efficacité d'un éventuel retour à une synchronisation convenable des cavités gauches.

On notera également que ce mode de stimulation peut interférer sur le remplissage des cavités droites : en effet, du fait de la stimulation prématurée de l'oreillette gauche, la synchronisation OD-VD est significativement modifiée, d'une manière qui peut être incompatible avec un remplissage satisfaisant du ventricule droit (on explicitera ces aspects dans la description détaillée). La thérapie d'amélioration du remplissage du ventricule gauche est donc susceptible d'induire des effets délétères sur le remplissage du ventricule droit, donc du côté qui n'était pas affecté par la pathologie que l'on cherche à traiter.

Au surplus, cette technique requiert la présence d'une sonde de détection ventriculaire droite, de manière à s'assurer que l'*overdriving* n'est contrôlé que sur la base de l'activité auriculaire réelle, et non sur des signaux détectés au niveau de l'oreillette mais provenant en fait de la dépolarisation des ventricules (signaux *far-field*).

Une autre technique est décrite dans le EP 2 471 575 A1 (Sorin CRM SAS) qui, notamment pour pallier ces inconvénients, met en oeuvre un capteur de recueil d'un signal d'accélération endocardiaque (EA). Le signal EA est analysé de manière à y détecter la présence d'une composante spécifique reflétant la contraction auriculaire (composante EA4) et à identifier l'instant d'apparition de cette composante. Si la composante EA4 est présente, ceci signifie que le séquencement des contractions auriculaires est correct, car dans le cas contraire (contractions auriculaires gauches trop tardives), la composante EA4 se trouverait noyée dans la composante du signal EA correspondant à la contraction ventriculaire immédiatement consécutive (composante EA1). L'intervalle de stimulation interauriculaire (intervalle AA) est alors ajusté dynamiquement en fonction du résultat de cette analyse.

Cette technique impose cependant de disposer d'une sonde implantée pourvue d'un capteur d'accélération endocardiaque, ainsi que d'un générateur susceptible de traiter les signaux EA délivrés par un tel capteur.

L'un des buts de l'invention est de proposer une nouvelle technique de traitement de l'insuffisance cardiaque à fraction d'éjection préservée chez les patients souffrant d'un délai interauriculaire mécanique, qui pallie les inconvénients des méthodes proposées jusqu'à présent, et qui ne nécessite pas de recourir à des moyens de recueil et d'analyse d'un signal EA.

Un autre but de l'invention est de proposer une telle technique qui assure un rétablissement de la fonction diastolique de manière à la fois simple (en termes de moyens mis en oeuvre) et très réactive (efficacité obtenue cycle à cycle).

L'invention opère par une stimulation auriculaire exclusive permettant de rétablir un séquencement satisfaisant OG-VG (séquencement de la contraction de l'oreillette gauche par rapport à celle du ventricule gauche), de manière que l'oreillette puisse remplir correctement sa fonction d'achèvement du remplissage du ventricule gauche.

On notera que pour mettre en oeuvre l'invention il n'est pas besoin de disposer de moyens de stimulation ventriculaire et/ou de recueil des dépolarisations ventriculaires. Ces derniers peuvent certes exister, par exemple dans le cas d'un stimulateur multisite assurant, outre la stimulation des oreillettes, celle du ventricule droit ou même celle des deux ventricules (stimulateurs triple ou quadruple chambre). Mais ces moyens ventriculaires ne sont pas impliqués dans le traitement de l'insuffisance cardiaque à fraction d'éjection préservée, qui vise à pallier un trouble préexistant de conduction entre les oreillettes, à savoir un bloc interauriculaire se traduisant par un retard excessif de conduction entre l'oreillette droite et l'oreillette gauche.

Plus précisément, l'invention propose un dispositif comprenant, de manière en elle-même connue d'après le EP 2 471 575 A1 précité, des circuits à processeur numérique configurés de manière à opérer :

- le recueil de dépolarisations auriculaires droites ;
- le recueil de dépolarisations auriculaires gauches ;
- la délivrance d'impulsions de stimulation auriculaire gauche ; et
- la délivrance, sélectivement sur la base de paramètres de contrôle ajustables, desdites impulsions de stimulation auriculaires gauches de manière prématurée, par application d'un couplage interauriculaire abrégé, plus court que l'intervalle de couplage du rythme sinusal.

De façon caractéristique de l'invention, le dispositif est dépourvu de moyens de recueil et d'analyse de l'accélération endocardiaque, et le processeur numérique est configuré de manière à :

- après un premier cycle cardiaque sans stimulation auriculaire gauche, délivrer une impulsion de stimulation auriculaire gauche au cours d'un deuxième cycle cardiaque immédiatement consécutif, avec application d'un couplage interauriculaire,
  ledit couplage interauriculaire appliqué au cours du deuxième cycle cardiaque immédiatement consécutif étant un couplage abrégé plus court que l'intervalle de couplage du rythme sinusal, de sorte que ladite impulsion de stimulation auriculaire gauche délivrée au cours de ce deuxième cycle cardiaque soit une impulsion prématurée ;
- délivrer une impulsion de stimulation auriculaire gauche non prématurée au cours d'un troisième cycle cardiaque immédiatement consécutif audit deuxième cycle cardiaque, avec application d'un couplage interauriculaire correspondant à l'intervalle de couplage du rythme sinusal, de manière à :

  • évaluer l'intervalle de couplage auriculaire droit séparant les dépolarisations auriculaires droites auxdits deuxième et troisième cycles cardiaques ;
  • comparer l'intervalle de couplage auriculaire droit ainsi évalué à l'intervalle de couplage du rythme sinusal ; et
  • modifier, ou non, au moins l'un desdits paramètres de contrôle ajustables en fonction du résultat de la comparaison.

Selon diverses caractéristiques subsidiaires avantageuses :

- le paramètre de contrôle modifié ou non en fonction du résultat de la comparaison est le couplage interauriculaire abrégé ;
- dans ce cas, si l'intervalle de couplage auriculaire droit est inférieur à l'intervalle de couplage du rythme sinusal le processeur numérique est en outre configuré de manière à modifier d'un pas de variation ledit couplage interauriculaire abrégé, puis délivrer une impulsion de stimulation auriculaire gauche prématurée au cours d'un cycle cardiaque consécutif, avec application dudit couplage interauriculaire abrégé modifié d'un pas ;

- sur détection d'un évènement prédéterminé, le processeur numérique est alors configuré de manière à : inhiber la délivrance de toute impulsion de stimulation auriculaire gauche au cours d'au moins un cycle cardiaque ; mesurer sur au moins un cycle cardiaque l'intervalle d'échappement auriculaire et le délai interauriculaire ; et mémoriser cet intervalle d'échappement en tant qu'intervalle de couplage du rythme sinusal pour la détermination dudit couplage interauriculaire abrégé et dudit délai interauriculaire correspondant à l'intervalle de couplage du rythme sinusal, au cours des cycles ultérieurs ;
- l'évènement prédéterminé est l'expiration d'un intervalle de temps fixe prédéterminé ;
- l'évènement prédéterminé est la détection du dépassement d'un seuil prédéterminé par la variation de l'intervalle de couplage auriculaire.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 illustre de façon schématique la position des différents sites impliqués dans l'activité électrique cyclique, spontanée ou stimulée, du coeur.

La Figure 2 est une série de chronogrammes illustrant différents signaux caractérisant l'activité cardiaque au cours de deux cycles successifs, dans le cas d'une technique d'*overdriving* connue.

La Figure 3 est un diagramme illustrant, dans un exemple de réalisation, le séquencement des détections et des stimulations auriculaires et ventriculaires sur quatre cycles cardiaques successifs, dans le cas d'un dispositif fonctionnant conformément aux enseignements de l'invention.

La Figure 4 est un organigramme décrivant la manière de délivrer la stimulation cardiaque suivant un exemple de réalisation de l'invention.

La Figure 5 est un schéma par blocs illustrant un exemple de réalisation d'un dispositif médical implantable pouvant être utilisé pour mettre en oeuvre différentes caractéristiques présentées dans la présente description.

On va maintenant décrire un exemple de réalisation du dispositif de l'invention.

En ce qui concerne ses aspects logiciels, l'invention peut être mise en oeuvre par une programmation appropriée d'algorithmes de commande exécutés par un microcontrôleur ou processeur numérique de signal d'un stimulateur connu, par exemple de type stimulateur cardiaque, resynchroniseur et/ou défibrillateur, comprenant des moyens d'acquisition d'un signal fourni par des sondes endocavitaires.

L'invention peut notamment être appliquée aux dispositifs implantables tels que ceux des familles *Reply, Ovatio* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

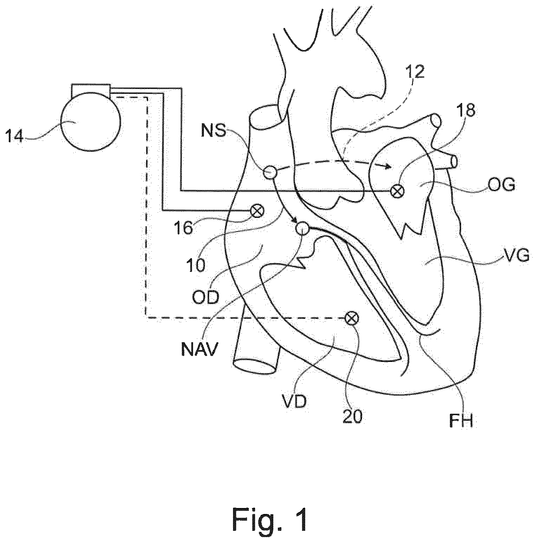

La Figure 1 illustre de façon schématique le coeur avec ses quatre cavités : oreillette droite OD, ventricule droit VD, oreillette gauche OG et ventricule gauche VG.

La contraction coordonnée des différentes cavités prend sa source au noeud sinusal NS, puis l'onde de dépolarisation est conduite au noeud atrioventriculaire NAV (conduction schématisée par la flèche 10), puis à partir de ce noeud au faisceau de His FH, et enfin aux tissus des ventricules droit et gauche VD et VG, provoquant la contraction de ceux-ci.

Par ailleurs, l'onde de dépolarisation issue du noeud sinusal NS provoque la contraction de l'oreillette droite OD puis, après conduction interauriculaire (conduction schématisée par la flèche 12) jusqu'à l'oreillette gauche OG, provoque la contraction de cette dernière.

Dans le cas d'un patient souffrant d'insuffisance cardiaque à fraction d'éjection préservée, la conduction atrioventriculaire (flèche 10, du noeud sinusal NS au noeud atrioventriculaire NAV) est généralement préservée, de même que les voies de conduction permettant d'assurer une contraction synchrone des deux ventricules droit VD et gauche VG.

En revanche, lorsque la conduction interauriculaire (flèche 12) est altérée, cela entraine un retard de la dépolarisation et donc de la contraction de l'oreillette gauche OG par rapport aux ventricules. Ceci induit un mauvais séquencement de la contraction de l'oreillette gauche OG par rapport à celle du ventricule gauche VG, avec une contraction plus ou moins concomitante de ces deux cavités. De ce fait, l'oreillette gauche OG ne peut plus remplir correctement sa fonction, qui est de terminer le remplissage du ventricule gauche VG.

Le dispositif implanté comporte un générateur 14 relié à une électrode 16 de stimulation/détection au niveau de l'oreillette droite OD, et à une électrode 18 de stimulation/détection au niveau de l'oreillette gauche OG.

L'électrode 18 peut être notamment positionnée dans le sinus coronaire ou dans une veine tributaire du réseau coronaire telle que la veine de Marshall, ou bien sur le septum interauriculaire, ou encore directement dans l'oreillette gauche après ponction du septum interauriculaire.

Dans une forme de mise en oeuvre particulière décrite à la fin de la présente description, ou bien à d'autres fins que la mise en oeuvre de l'invention, le dispositif peut également comporter une sonde ventriculaire 20 telle qu'une sonde endocavitaire logée dans le ventricule droit VD.

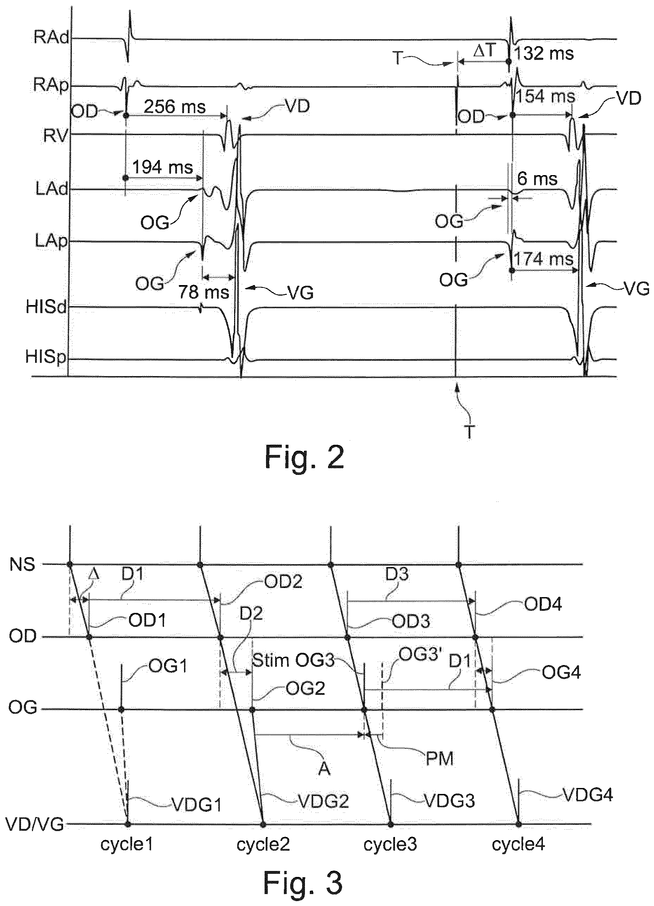

On a illustré sur la Figure 2 la technique conventionnelle *d'overdriving* habituellement mise en oeuvre avec ce type de dispositif pour remédier à une insuffisance à fraction d'éjection préservée.

On a représenté sur cette figure les tracés de divers enregistrements recueillis au cours de deux cycles cardiaques successifs - le premier en rythme spontané, le second avec *overdriving.* Ces enregistrements correspondent aux dérivations suivantes : électrodes distale et proximale sur l'oreillette droite (RAd et RAp), ventricule droit (RV), électrodes distale et proximale sur l'oreillette gauche (LAd et LAp), et électrodes distale et proximale sur le faisceau de His (HISd et HISp), correspondant plus ou moins à la dépolarisation du ventricule gauche (notamment la dérivation HISd).

Au premier cycle cardiaque on constate un retard, conforme, de 256 ms entre la dépolarisation spontanée du ventricule droit (VD) et la dépolarisation de l'oreillette droite (OD). En revanche, le retard OD-OG entre les dépolarisations respectives des deux oreillettes présente une valeur, excessive et pathologique, de 194 ms, ce qui a pour conséquence de ne laisser qu'un intervalle de 78 ms entre la dépolarisation de l'oreillette gauche (OG) et la contraction du ventricule gauche (VG), intervalle trop bref pour permettre un remplissage satisfaisant de ce ventricule gauche. Dans certains cas extrêmes, la contraction de l'oreillette gauche et celle du ventricule gauche peuvent même être quasi-concomitantes, réduisant à néant le rôle de l'oreillette dans le remplissage du ventricule.

Au second cycle cardiaque, qui est un cycle avec *overdriving,* le dispositif stimule l'oreillette gauche à un instant donné (T) présentant une prématurité délibérée $\Delta T$ par rapport au rythme sinusal normal correspondant à l'intervalle de couplage OD-OD d'un cycle au suivant. Cette stimulation prématurée de l'oreillette gauche permet de rétablir un séquencement OG-VG pratiquement physiologique (174 ms) ; en revanche, le séquencement OD-VD est significativement modifié, puisque ce délai est maintenant de 154 ms au lieu de 256 ms, ce qui peut avoir pour conséquence des effets délétères en ce qui concerne le régime hémodynamique côté droit.

La Figure 3 est un diagramme illustrant le séquencement des détections et des stimulations auriculaires et ventriculaires sur quatre cycles cardiaques successifs, dans le cas d'un dispositif fonctionnant conformément aux enseignements de l'invention.

On a représenté sur ce diagramme divers marqueurs correspondant :

- au rythme sinusal NS,
- au rythme auriculaire droit OD,
- au rythme auriculaire gauche OG, et
- au rythme ventriculaire VD/VG (on fera l'hypothèse qu'il n'y a pas de délai entre la dépolarisation du ventricule droit et celle du ventricule gauche).

Le principe de base de l'invention consiste à commander le dispositif avec une stimulation de l'oreillette gauche contrôlée de manière à éviter tout effet d'*overdriving* et ainsi ne pas modifier la séquence atrioventriculaire droite.

Initialement (Cycle 1), l'influx initial né dans le noeud sinusal se propage dans l'oreillette droite : le marqueur OD1 correspond à la détection par la sonde auriculaire droite, avec un retard $\Delta$ par rapport au début du cycle, retard correspondant au délai de propagation jusqu'à l'oreillette droite depuis le noeud sinusal.

Cet influx dépolarise ensuite le noeud atrioventriculaire et atteint les ventricules (marqueur VDG1). Il se propage également vers l'oreillette gauche (marqueur OG1), avec un retard important dans la pathologie d'insuffisance à fraction d'éjection préservée, de sorte que dans l'exemple illustré, la dépolarisation de l'oreillette gauche (marqueur OG1) et celle des ventricules (marqueur VDG1) sont pratiquement concomitantes.

Le cycle se répète (Cycle 2), avec un intervalle de couplage auriculaire D1 correspondant au rythme sinusal. Le défaut de séquencement de l'oreillette gauche se reproduit, avec un intervalle D2 trop long entre les dépolarisations des oreillettes à droite (marqueur OD2) et à gauche (marqueur OG2).

Au cycle suivant (Cycle 3), conformément à l'invention, une stimulation est délivrée à l'oreillette gauche (marqueur StimOG3) après une durée A par rapport à la précédente dépolarisation de l'oreillette gauche (marqueur OG2). Cette durée est choisie pour engendrer une prématurité PM prédéterminée (par rapport au marqueur OG3' de la dépolarisation que l'on aurait eue avec l'intervalle de couplage auriculaire normal), calculée de manière à :

- s'assurer que l'intervalle OG3-VDG3 est suffisamment long pour établir un séquencement normal des cavités gauches, et
- ne pas modifier le séquencement OD3-VDG3 des cavités droites.

Pour ce faire, le dispositif détermine les durées D1 (OD1-OD2) et D2 (OD2-OG2) au cours des cycles n°1 et 2, et calcule une prématurité :

$$PM = D2\text{-}a$$

a étant un intervalle de temps choisi pour correspondre par exemple au délai de propagation entre le noeud sinusal et l'oreillette droite (délai schématisé par la flèche 10 sur la Figure 1), de manière que la dépolarisation de l'oreillette gauche ne soit pas synchrone de celle de l'oreillette droite, mais soit légèrement retardée par rapport à cette dernière. Ce délai a peut être fixe, par exemple de 50 ms, ou programmable. En variante, a peut être nul si l'on veut que les deux oreillettes soient synchrones, ou même négatif si l'on veut que la contraction de l'oreillette gauche précède celle de l'oreillette droite.

Cette prématurité PM correspond, comme expliqué plus haut, à une durée A=D1-PM comptée à partir de la précédente dépolarisation de l'oreillette gauche (marqueur OG2) avant de délivrer une stimulation à l'oreillette gauche (marqueur StimOG3).

Si une période de *blanking* post-stimulation auriculaire gauche est appliquée, on privilégiera un intervalle a positif qui positionnera la stimulation auriculaire gauche juste après la détection auriculaire droite, évitant ainsi que l'application d'un blanking post-stimulation ne compromette cette détection.

Au cycle suivant (Cycle 4) le dispositif délivre à l'oreillette gauche une stimulation (marqueur StimOG4), mais avec un intervalle interauriculaire D1 correspondant à l'intervalle OD1-OD2 qui avait été mesuré au cours des cycles précédents avant que l'oreillette gauche ne soit stimulée.

Le dispositif mesure alors l'intervalle OD3-OD4 (intervalle D3) et compare cet intervalle à l'intervalle OD1-OD2 (intervalle D1) :

- si D3 = D1, ceci signifie que le couplage auriculaire est conservé, et que la stimulation (prématurée) de l'oreillette gauche au cycle précédent n'a pas provoqué de dépolarisation indirecte de l'oreillette droite ;
- si D3 < D1, ceci signifie que la prématurité est trop importante, et a modifié la dépolarisation de l'oreillette droite - ce que l'on veut précisément éviter.
La stimulation de l'oreillette gauche sera alors suspendue pendant un (ou plusieurs) cycle(s), et la séquence de stimulation sera ensuite reprise, mais en réduisant la prématurité d'un pas de variation (c'est-à-dire en allongeant la durée A), et ainsi de suite si nécessaire jusqu'à ce que l'on trouve une séquence n telle que l'intervalle

$$ODn-ODn+1 = OD1-OD2.$$

De façon régulière, par exemple toutes les minutes, ou en cas de variation significative de l'intervalle de couplage auriculaire droit (modification de l'intervalle D1 dépassant un seuil donné), le processus ci-dessus est réitéré, de manière à disposer d'un cycle sans stimulation de l'oreillette gauche, qui servira de référence.

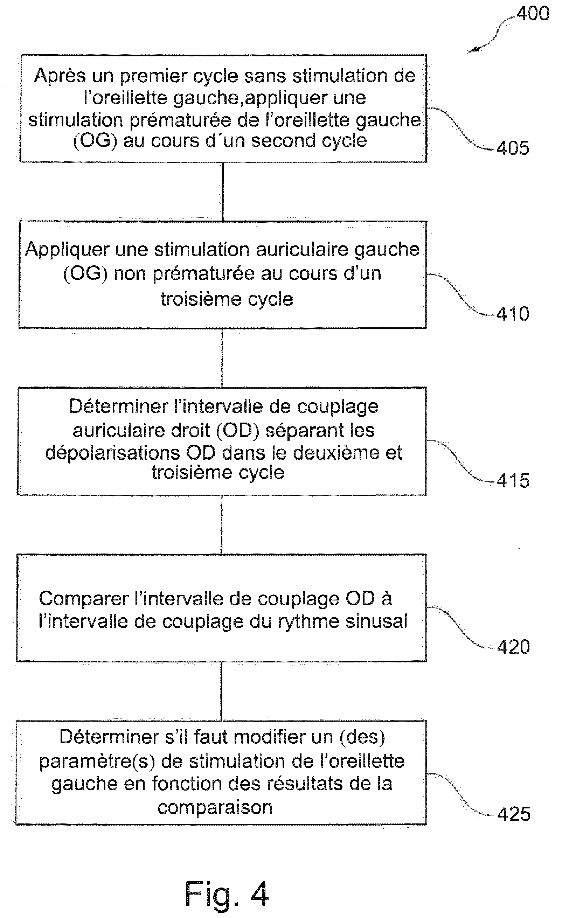

On a représenté sur la Figure 4 un schéma de principe d'un organigramme 400 de délivrance de la stimulation cardiaque selon un exemple de réalisation de l'invention.

Après un premier cycle cardiaque au cours duquel aucune stimulation auriculaire gauche OG n'est délivrée, le dispositif (par exemple, un dispositif médical implantable) délivre une impulsion de stimulation OG prématurée lors du deuxième cycle cardiaque (étape 405), qui suit immédiatement le premier cycle cardiaque. La stimulation OG prématurée est ajustée de façon à entraîner l'application d'un couplage interauriculaire abrégé plus court que l'intervalle de couplage du rythme sinusal.

Le dispositif applique ensuite une impulsion de stimulation OG non prématurée au cours d'un troisième cycle cardiaque (étape 410), qui suit immédiatement le deuxième cycle cardiaque. L'impulsion de stimulation OG non prématurée est ajustée de façon à entraîner l'application d'un couplage interauriculaire qui correspond à l'intervalle de couplage de la période sinusale.

Le dispositif détermine ensuite un intervalle de couplage auriculaire droit OD séparant les dépolarisations de l'oreillette droite lors des deuxième et troisième cycles (étape 415) . Le dispositif compare l'intervalle de couplage OD à l'intervalle de couplage du rythme sinusal (étape 420). En fonction du résultat de la comparaison, le dispositif détermine s'il y a lieu et/ou comment modifier un ou plusieurs paramètres de la stimulation de l'oreillette gauche appliquée lors des cycles cardiaques ultérieurs (étape 425). Une ou plusieurs étapes de l'organigramme 400 peuvent être répétées à la suite de la modification des paramètres, pour modifier davantage les paramètres et/ou optimiser la stimulation de l'oreillette gauche.

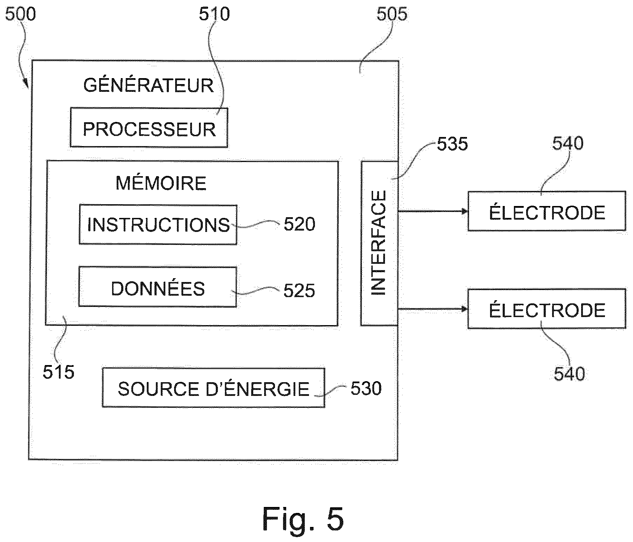

On a représenté sur la Figure 5 un schéma de principe d'un exemple de réalisation d'un dispositif 500 susceptible d'être utilisé pour mettre en oeuvre différentes fonctionnalités décrites ci-dessus.

Le dispositif 500 peut être un dispositif médical implantable conçu pour être implanté chez un sujet, par exemple un patient. Le dispositif 500 comprend un générateur 505 configuré pour générer des signaux de stimulation de tissus, par exemple un tissu cardiaque, un tissu nerveux, etc.). Des signaux de détection peuvent également être reçus en provenance d'une ou plusieurs électrodes 540 couplées au générateur 505 via une interface 535 filaire ou sans fil. Dans certains modes de réalisation, le générateur 505 peut être connecté à des électrodes 540 par des câbles. Également, dans certains modes de réalisation le générateur 505 peut être connecté à des électrodes et inclure une source d'énergie 530 telle qu'une batterie. Dans certains modes de réalisation, le générateur 505 peut comprendre un ou plusieurs émetteurs-récepteurs sans fil configurés pour permettre au générateur 505 de communiquer sans fil avec un ou plusieurs autres dispositifs (par exemple, des dispositifs externes au patient). Par exemple, un émetteur-récepteur sans fil peut communiquer avec un dispositif informatique d'usage général ou à usage spécial (par exemple, un ordinateur de bureau, une tablette, etc.), notamment dans un hôpital ou un environnement clinique pour transmettre des signaux vers et depuis le dispositif informatique externe, par exemple recevoir des signaux de commande configurés pour commander les paramètres de détection/stimulation du générateur 505 et/ou fournir des données relatives à la détection/stimulation au dispositif informatique externe.

Le générateur 505 comprend un processeur 510 et une mémoire 515. Le processeur 510 peut être de n'importe quel type standard ou être un processeur pour usages spéciaux susceptible d'être intégré dans le boitier du générateur 505. La mémoire 515 peut inclure tout type de support approprié de stockage lisible par machine pour stocker des instructions exécutables par machine 520 et/ou d'autres données 525. Les instructions 520 peuvent être exécutées par le processeur 510 pour mettre en oeuvre diverses opérations décrites dans le présent exposé. À titre d'exemple, de tels supports de stockage lisibles par une machine peuvent inclure des mémoires RAM, ROM, EPROM, EEPROM , mémoire flash ou tout autre moyen qui peut être utilisé pour transférer ou stocker du code de programme sous la forme d'instructions exécutables par machine ou de structures de données et qui peut être lu par une machine avec un processeur. Toute combinaison de ce qui précède est également incluse dans le champ d'application de ce que l'on désigne comme étant un support de stockage lisible par machine. Les machines ou supports de stockage lisibles par ordinateur mentionnés ici ne comprennent pas les supports temporaires, par exemple les signaux en champ libre.

Dans certains modes de réalisation, la mémoire 515 peut comporter un ou plusieurs modules avec des instructions configurées pour amener le processeur 510 à réaliser diverses fonctions telles que celles décrites précédemment. Par exemple, la mémoire 515 peut comprendre un module de stimulation configuré pour commander la génération et/ou la transmission d'impulsions de stimulation (par exemple, les impulsions de stimulation auriculaire) à des électrodes 540. La mémoire 515 peut comprendre un module de détection configuré pour détecter les informations collectées par les électrodes 540 et évaluer la réponse à fournir (par exemple, l'évaluation de l'intervalle de couplage auriculaire droit et/ou la comparaison de l'intervalle de couplage auriculaire droit et l'intervalle du rythme sinusal). Le module de détection peut être configuré de manière à modifier un ou plusieurs paramètres de la stimulation en fonction de cette évaluation. Les paramètres peuvent également être stockés dans la mémoire 515.

## Revendications

1. Un dispositif médical implantable actif du type prothèse cardiaque de stimulation, resynchronisation et/ou défibrillation,

ce dispositif comprenant :

- une première électrode de stimulation/détection (16), destinée à être implantée au niveau de l'oreillette droite (OD) ;
- une seconde électrode de stimulation/détection (18), destinée à être implantée au niveau de l'oreillette gauche (OG) ;
- un générateur (14), apte à assurer :

- la délivrance sur ladite seconde électrode de stimulation/détection (18) d'impulsions de stimulation auriculaire gauche (StimOG) ; et
- la délivrance, sélectivement sur la base de paramètres de contrôle ajustables, desdites impulsions de stimulation auriculaires gauches de manière prématurée, par application d'un couplage interauriculaire abrégé, plus court que l'intervalle de couplage du rythme sinusal,

**caractérisé en ce que** le dispositif est dépourvu de moyens de recueil et d'analyse de l'accélération endocardiaque,
et **en ce qu'**il comprend un processeur numérique configuré pour piloter le générateur de manière à :

- après un premier cycle cardiaque sans stimulation auriculaire gauche (Cycle 2), délivrer une impulsion de stimulation auriculaire gauche au cours d'un deuxième cycle cardiaque immédiatement consécutif (Cycle 3), avec application d'un premier couplage interauriculaire (A), ledit premier couplage interauriculaire (A) appliqué au cours du deuxième cycle cardiaque immédiatement consécutif étant un couplage abrégé plus court que l'intervalle de couplage du rythme sinusal (D1), de sorte que ladite impulsion de stimulation auriculaire gauche délivrée au cours de ce deuxième cycle cardiaque soit une impulsion prématurée ;
- délivrer une impulsion de stimulation auriculaire gauche non prématurée au cours d'un troisième cycle cardiaque (Cycle 4) immédiatement consécutif audit deuxième cycle cardiaque (Cycle 3), avec application d'un second couplage interauriculaire (D2) correspondant à l'intervalle de couplage du rythme sinusal (D1),

et **en ce que** le processeur numérique est configuré de manière à :

· évaluer l'intervalle de couplage auriculaire droit (D3) séparant les dépolarisations auriculaires droites auxdits deuxième et troisième cycles cardiaques ;
· comparer l'intervalle de couplage auriculaire droit (D3) ainsi évalué à l'intervalle de couplage du rythme sinusal (D1) ; et

· modifier, ou non, au moins l'un desdits paramètres de contrôle ajustables en fonction du résultat de la comparaison.

2. Le dispositif de la revendication 1, dans lequel ledit paramètre de contrôle modifié ou non en fonction du résultat de la comparaison est le couplage interauriculaire abrégé.

3. Le dispositif de la revendication 2, dans lequel, si l'intervalle de couplage auriculaire droit (D3) est inférieur à l'intervalle de couplage du rythme sinusal (D1) le processeur numérique est en outre configuré de manière à :

modifier d'un pas de variation ledit couplage interauriculaire abrégé ; puis

• délivrer une impulsion de stimulation auriculaire gauche prématurée au cours d'un cycle cardiaque consécutif, avec application dudit couplage interauriculaire abrégé modifié d'un pas.

4. Le dispositif de la revendication 1, dans lequel, sur détection d'un évènement prédéterminé, le processeur numérique est en outre configuré de manière à :

inhiber la délivrance de toute impulsion de stimulation auriculaire gauche au cours d'au moins un cycle cardiaque ;

mesurer sur au moins un cycle cardiaque l'intervalle d'échappement auriculaire et le délai interauriculaire ; et

mémoriser cet intervalle d'échappement en tant qu'intervalle de couplage du rythme sinusal pour la détermination dudit couplage interauriculaire abrégé et dudit délai interauriculaire correspondant à l'intervalle de couplage du rythme sinusal, au cours des cycles ultérieurs.

5. Le dispositif de la revendication 4, dans lequel ledit évènement prédéterminé est l'expiration d'un intervalle de temps fixe prédéterminé.

6. Le dispositif de la revendication 4, dans lequel ledit évènement prédéterminé est la détection du dépassement d'un seuil prédéterminé par la variation de l'intervalle de couplage auriculaire.

**Patentansprüche**

1. Implantierbare aktive medizinische Vorrichtung vom Typ Herzprothese zur Stimulation, Resynchronisation und/oder Defibrillation,

wobei diese Vorrichtung Folgendes umfasst:

- eine erste Stimulations-/Erfassungselektrode (16), die dazu ausgebildet ist, am rechten Vorhof (OD) implantiert zu werden;
- eine zweite Stimulations-/Erfassungselektrode (18), die dazu ausgebildet ist, am linken Vorhof (OD) implantiert zu werden;
- einen Generator (14), der in der Lage ist:

- die Abgabe von Impulsen zur Stimulation des linken Vorhofs (StimOG) durch die zweite Stimulations-/Erfassungselektrode (18); und
- selektiv auf der Grundlage von einstellbaren Kontrollparametern, die Abgabe der Impulse zur Stimulation des linken Vorhofs vorzeitig, unter Anwendung einer verkürzten interatrialen Kopplung, die kürzer als das Kopplungsintervall des Sinusrhythmuses ist,

zu gewährleisten,

**dadurch gekennzeichnet, dass** die Vorrichtung keine Mittel zur Aufnahme und Analyse der endokardialen Beschleunigung aufweist,
und dass sie einen digitalen Prozessor aufweist, der dazu konfiguriert ist, den Generator derart zu steuern, dass er:

- nach einem ersten Herzzyklus ohne Stimulation des linken Vorhofs (Zyklus 2) während eines unmittelbar darauffolgenden zweiten Herzzyklus (Zyklus 3), unter Anwendung einer ersten interatrialen Kopplung, einen Impuls zur Stimulation des linken Vorhofs abgibt, wobei die während des unmittelbar darauffolgenden zweiten Herzzyklus angewandte erste interatriale Kopplung (A) eine kürzere verkürzte Kopplung als das Kopplungsintervall des Sinusrhythmus (D1) ist, so dass der während dieses zweiten Herzzyklus abgegebene Impuls zur Stimulation des linken Vorhofs ein vorzeitiger Impuls ist;
- während eines auf den zweiten Herzzyklus (Zyklus 3) unmittelbar folgenden dritten Herzzyklus (Zyklus 4), unter Anwendung einer dem Kopplungsintervall des Sinusrhythmus (D1) entsprechenden zweiten interatrialen Kopplung (D2), einen nicht vorzeitigen Impuls zur Stimulation des linken Vorhofs abgibt,

und dass der digitale Prozessor derart konfiguriert ist, dass er:

• das Kopplungsintervall des rechten Vorhofs (D3), das die Depolarisationen des rechten Vorhofs trennt, im zweiten und dritten Herzzyklus bemisst;

• das derart bemessene Kopplungsintervall des rechten Vorhofs (D3) mit dem Kopplungsintervall des Sinusrhythmus vergleicht; und

• in Abhängigkeit des Ergebnisses des Vergleichs, mindestens einen der einstellbaren Kontrollparameter ändert oder nicht.

**2.** Vorrichtung nach Anspruch 1, bei der der in Abhängigkeit des Ergebnisses des Vergleichs geänderte oder nicht geänderte einstellbare Kontrollparameter die verkürzte interatriale Kopplung ist.

**3.** Vorrichtung nach Anspruch 2, bei der, wenn das Kopplungsintervall des rechten Vorhofs (D3) kleiner als das Kopplungsintervall des Sinusrhythmus (D1) ist, der digitale Prozessor weiter dazu konfiguriert ist:

• die verkürzte interatriale Kopplung um einen Variationsschritt zu ändern; und anschließend

• während eines darauffolgenden Herzzyklus, unter Anwendung der geänderten oder nicht geänderten interatrialen Kopplung, einen vorzeitige Impuls zur Stimulation des linken Vorhofs abzugeben.

**4.** Vorrichtung nach Anspruch 1, bei der, bei Erfassung eines vorbestimmten Ereignisses, der digitale Prozessor außerdem dazu ausgebildet ist:

• während mindestens einem Herzzyklus, die Abgabe eines Impulses zur Stimulation des linken Vorhofs zu hemmen;

• während mindestens einem Herzzyklus, das atriale Ausströmintervall und die interatriale Verzögerung zu messen; und

• dieses Ausströmintervall als Kopplungsintervall des Sinusrhythmus zu speichern, um während den darauffolgenden Zyklen die verkürzte interatriale Kopplung und die dem Kopplungsintervall des Sinusrhythmus entsprechende interatriale Verzögerung zu bestimmen.

**5.** Vorrichtung nach Anspruch 4, bei der das vorbestimmte Ereignis der Ablauf eines vorbestimmten festen Zeitintervalls ist.

**6.** Vorrichtung nach Anspruch 4, bei der das vorbestimmte Ereignis die Erfassung einer Überschreitung einer vorbestimmten Schwelle durch die Variation des atrialen Kopplungsintervalls ist.

**Claims**

**1.** An active implantable medical device of the cardiac stimulation, resynchronization and/or defibrillation prosthesis type, the device comprising:

- a first stimulation/detection electrode (16) to be implanted at the right atrium (OD);
- a second stimulation/detection electrode (18) to be implanted at the left atrium (OG);
- a generator (14) capable of ensuring:

- the delivery of left atrium stimulation pulses (StimOG) by said second stimulation/detection electrode (18); and
- selectively based on adjustable control parameters, the premature delivery of said left atrium stimulation pulses, while applying a shortened inter-atrial coupling that is shorter than the sinus rhythm coupling interval,

**characterized in that** the device is deprived of means for collecting and analyzing the endocardial acceleration,

and **in that** it comprises a digital processor configured to control the generator in such a manner that:

- after a first cardiac cycle without left atrium stimulation (Cyle 2), a left atrium stimulation pulse is delivered during an immediately subsequent second cardiac cycle (Cycle 3), while applying a first inter-atrial coupling (A),

said first inter-atrial coupling (A) applied during the immediately subsequent second cardiac cycle being a shortened coupling, that is shorter than the sinus rhythm coupling interval (D1), in order for said left atrium stimulation pulse delivered during this second cardiac cycle to be a premature pulse;

- a non premature left atrium stimulation pulse is delivered during a third cardiac cycle (Cycle 4) that is immediately subsequent to said second cardiac cycle (Cycle 3), while applying a second inter-atrial coupling (D2) corresponding to the sinus rhythm coupling interval (D1),

and **in that** the digital processor is configured so as to :

• evaluate the right atrial coupling interval (D3) separating the right atrial depolarizations in said second and third cardiac cycles;

• compare the right atrial coupling interval (D3) thus evaluated with the sinus rhythm coupling interval (D1) ; and

• modify or not at least one of said adjustable control parameters according to the result of the comparison.

**2.** The device according to claim 1, in which said control parameter that has been modified or not according to the result of the comparison is the shortened inter-atrial coupling.

**3.** The device according to claim 2, in which, if the right atrial coupling interval (D3) is smaller than the sinus rhythm coupling interval (D1), the digital processor is further configured to:

> • modify said shortened inter-atrial coupling by one variation step; and then
> • deliver a premature left atrium stimulation pulse during a subsequent cardiac cycle, while applying said shortened inter-atrial coupling that has been modified by one step.

**4.** The device according to claim 1, in which, upon detection of a predetermined event, the digital processor is further configured so as to:

> • inhibit the delivery of any left atrium stimulation pulse during at least one cardiac cycle;
> • measure during at least one cardiac cycle the atrial escape interval and the inter-atrial delay; and
> • memorize this escape interval as a sinus rhythm coupling interval for determining said shortened inter-atrial coupling and said inter-atrial delay corresponding to the sinus rhythm coupling interval during later cycles.

**5.** The device according to claim 4, in which said predetermined event is the expiry of a predetermined fixed time interval.

**6.** The device according to claim 4, in which said predetermined event is the detection of a predetermined threshold being exceeded by the variation of the atrial coupling interval.

Fig. 1

EP 2 873 436 B1

Fig. 2

Fig. 3

12

400

Après un premier cycle sans stimulation de l'oreillette gauche, appliquer une stimulation prématurée de l'oreillette gauche (OG) au cours d´un second cycle

405

Appliquer une stimulation auriculaire gauche (OG) non prématurée au cours d'un troisième cycle

410

Déterminer l'intervalle de couplage auriculaire droit (OD) séparant les dépolarisations OD dans le deuxième et troisième cycle

415

Comparer l'intervalle de couplage OD à l'intervalle de couplage du rythme sinusal

420

Déterminer s'il faut modifier un (des) paramètre(s) de stimulation de l'oreillette gauche en fonction des résultats de la comparaison

425

# Fig. 4

Fig. 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7474921 B1

- EP 2471575 A1